# EUROPEAN PATENT SPECIFICATION

(11) **EP 4 176 838 B1**
(45) Date of publication and mention of the grant of the patent: **23.04.2025**
(21) Application number: 21832804.5
(22) Date of filing: 05.07.2021
(51) Int. Cl.: A61F 2/07, A61F 2/06

(54) **COVERED STENT FOR IMPLANTATION AT VASCULAR BRANCH, AND COVERED STENT SYSTEM**
ABGEDECKTER STENT ZUR IMPLANTATION AN EINEM GEFÄSSZWEIG UND ABGEDECKTES STENTSYSTEM
ENDOPROTHÈSE RECOUVERTE POUR IMPLANTATION AU NIVEAU D'UNE BRANCHE VASCULAIRE ET SYSTÈME D'ENDOPROTHÈSE RECOUVERTE

(30) Priority: 03.07.2020 CN 202010638641
(43) Date of publication of application: 10.05.2023
(73) Proprietor: Forever Medical (Suzhou) Co., Ltd., Suzhou, Jiangsu 215000 (CN)
(72) Inventor: ZHANG, Xiaoming, Suzhou, Jiangsu 215000 (CN)
(74) Representative: Forresters IP LLP
(86) International application number: PCT/CN2021/104487
(87) International publication number: WO 2022/002274

(56) References cited:
- WO-A1-2016/008944
- CN-A- 105 030 372
- CN-A- 110 868 960
- CN-A- 112 022 435
- CN-U- 205 286 610
- CN-U- 208 525 133
- CN-U- 208 525 133
- CN-U- 212 346 807
- CN-U- 213 030 936
- US-A1- 2014 336 749

## Description

### TECHNICAL FIELD

The present application relates to the field of medical devices, and in particular, to a covered stent and a covered stent system for implantation at vascular branches.

### BACKGROUND ART

High blood pressure, connective tissue disease, and chest trauma may all lead to aortic lesions. Endovascular repair has become a reliable option for treating various aortic lesions. As aortic diseases involving branch vessels (such as aneurysms, arterial dissection, arterial penetrating ulcers, etc.) become more and more serious, and the importance of the reconstruction of blood supply of branch vessels is gradually discovered, the problem of how to effectively treat aortic lesions without affecting the blood supply of the branch vessels near the lesion has become the focus of attention in the interventional treatment operations for aortic lesions.

Currently, the clinical interventional techniques for treating aortic branch lesions mainly include a "chimney" technique, a branch stent technique, and an in-situ fenestration technique and the like. The main defects of the above three treatment techniques are: 1. A chimney stent will affect the adherence of a covered stent to the aorta, which is easy to cause endoleak. In addition, the aortic covered stent will compress the chimney stent, which may easily cause the cross section of the chimney stent channel to become smaller or occluded, and the long-term patency rate is low. 2. The positioning of branch stents is difficult, and the operation is difficult to perform and is time consuming; the alignment of the branch stents is incorrect, and the long-term patency rate of the branch vessels is low. 3. The main defect of the in-situ fenestration technique is: before membrane rupture and fenestration are performed, the membrane will completely block blood supply of the branch vessels, which may cause serious intraoperative complications and even lead to the death of a patient. In this case, extracorporeal circulation needs to be established in advance before the operation can be performed, which increases the complexity and operation time of the operation. In addition, when a balloon is used to dilate the window, it is easy to cause the membrane to tear in the direction of the weaving pattern, resulting in endoleak.

Therefore, according to the shortcomings of the existing treatment technology, it is of great significance to design a covered stent capable of retaining the blood flow of the branch vessels on the basis of treating aortic lesions.

WO 2016/008944 A1 discloses a vascular prosthesis system, comprising at least one vascular prosthesis for insertion into a blood vessel of a human body, the vascular prosthesis having a proximal end, a distal end, and an elongated hollow cylindrical base body extending between the proximal end and the distal end. The elongated hollow cylindrical base body has a longitudinal axis c and a circumference b, which has a vascular prosthesis material for forming a circumferentially substantially closed jacket, wherein the vascular prosthesis material has a fabric structure made of interwoven threads and with a mesh density. The vascular prosthesis has in the vascular prosthesis material at least one perforation region located between the proximal and distal ends, with at least one hole which has a diameter x which is selected from a range from 0.5 mm to 2 mm, and wherein the at least one hole has a closed circumferential edge.

CN 208525133U discloses a stent graft suitable for aortic dissection surgery, comprising a stent graft main body and a branch-covered stent graft fixed on the main body of the stent graft during operation. The stent graft main body comprises a tubular main membrane and a main stent fixedly supporting the main membrane and having a tubular shape, the branch stent comprising a branch stent and a branching membrane covering the branch stent. A plurality of perforations can be pierced at the time of surgery to allow the main body of the stent graft to communicate with the inside of the branch stent graft.

### SUMMARY OF THE INVENTION

The purpose of the present application is to provide a covered stent and a covered stent system for vascular branches, which will not block the blood flow of nearby branch vessels while treating aortic lesions.

An object of the present application is achieved through the following technical solutions:
A covered stent for implantation at vascular branches, in accordance with claim 1.

Diameters of the micropores are in the range of 0.1mm to 0.6mm.

The membrane is provided with one or more microporous areas, and each of the one or more microporous areas are arranged in a range of 15 degrees to 360 degrees along the circumferential direction of the covered stent.

**In** one embodiment, a length of each of the one or more microporous areas is in the range of 3mm to 100mm.

In one embodiment, the stent body is formed by weaving or laser cutting.

In a preferred embodiment, the stent body has sufficient supporting force, and the shape of the stent body is a straight cylinder structure or a tapered structure.

In a preferred embodiment, the stent body is partially covered, and the stent at the proximal end portion of the stent body is uncovered.

In a preferred embodiment, the stent body is fully covered.

In a preferred embodiment, the material of the stent body includes, but is not limited to, nickel-titanium alloy, cobalt-chromium alloy, magnesium alloy, 316L stainless steel, metal tantalum, polymer materials, and the like.

A plurality of development marks are provided on the edge of each of the one or more microporous areas to indicate the position of the respective microporous area.

In a preferred embodiment, the development marks are attached to the edge of the respective microporous area by precision sewing or riveting.

In a preferred embodiment, the shapes of the development marks include, but are not limited to, circles, ovals, "8" shapes, and the like.

In a preferred embodiment, the number of the development marks is 2-10.

The material of the development marks includes, but is not limited to, platinum, gold, tantalum metal, medical stainless steel, and the like.

In one embodiment, the membrane is biocompatible, and the material of the membrane includes, but is not limited to, polytetrafluoroethylene, polyester, polyethylene terephthalate, polyurethane esters, and the like.

Compared with the prior art, the present application has the following advantages:
1. The covered stent and covered stent system provided by the present application are provided with one or more microporous areas on the membrane, and each of the one or more microporous areas have a plurality of micropores, which are capable of allowing blood flow to pass through; during the implantation of the covered stent, each of the one or more microporous areas can identify the vascular branches area and temporarily supply blood to the vascular branches; moreover, a plurality of micropores arranged in each of the one or more microporous areas are arranged into an array with pore spacing in the range of 0.2 mm to 2.0 mm, and the diameters of the micropores in the range of 0.1 mm to 0.6 mm; the setting of the micropore diameters and pore spacing can not only ensure the blood supply of the branch vessels, but also prevent the membrane from tearing during the puncture of the membrane breaker; even if the dilation balloon is further dilated, it only changes the size of the adjacent micropores in the respective microporous area, but will not tear the membrane; therefore, each of the one or more microporous areas can provide a good access indication and access window for the implantation of branch stents.
2. In the present application, a plurality of development marks are arranged on the edge of each of the one or more microporous areas to indicate the position of the respective microporous area. The microporous area section of the covered stent can be placed at the openings of the branch vessels through the development marks under the monitoring of Digital Subtraction Angiography (DSA), so that the covered stent will not block the blood flow of the branch vessels while treating aortic lesions; moreover, the development marks are also beneficial to the positioning of the branch stents, which can be released at the target position after passing through each of the one or more microporous areas to establish a blood supply channel of the vascular branches.
3. The diameters of the micropores in the present application are in the range of 0.1 mm to 0.6 mm, and micropores that are not located at the opening of the vascular branches are attached to the blood vessel walls; due to the limitation of the micropore diameter and the characteristics of high blood flow rate and high pressure in the aorta, after a period of time, blood accumulation will appear in the micropores, causing the micropores to be permanently closed, so there is no need to worry about endoleak.

### BRIEF DESCRIPTION OF DRAWINGS

FIG. 1 is a schematic diagram of an embodiment of treating aortic lesions using the "chimney" technique in the prior art.
FIG. 2 is a schematic diagram of the structure of a covered stent for treating aortic lesions using the branch stent technique in the prior art.
FIG. 3 is a schematic diagram of the structure of a covered stent for treating aortic lesions using the in-situ fenestration technique in the prior art.
Fig. 4a is a schematic diagram of the structure of a membrane breaker in the present application.
Fig. 4b is a schematic diagram of the structure of a dilation balloon in the present application.
Fig. 4c is a schematic diagram of the structure of a branch stent in the present application.
FIG. 4d is a schematic diagram of the structure of a guide wire in the present application.
FIG. 4e is a schematic diagram of the structure of a delivery system in the prior art.
FIG. 5a-5d are schematic diagrams illustrating one embodiment of the structure of a covered stent applicable to a vascular branch according to Embodiment 1 of the present application.
FIG. 6a-6c are schematic diagrams illustrating another embodiment of the structure of a covered stent applicable to a vascular branch according to Embodiment 1 of the present application.
FIG. 7a-7d are schematic diagrams illustrating the operation process of a covered stent system applicable to a vascular branch according to Embodiment 1 of the present application.
FIG. 8a-8d are schematic diagrams illustrating one embodiment of the structure of a covered stent applicable to vascular branches according to Embodiment 2 of the present application.
FIG. 9a-9d are schematic diagrams illustrating the operation process of a covered stent system applicable to vascular branches according to Embodiment 2 of the present application.
FIG. 10a-10d are schematic diagrams illustrating one embodiment of the structure of a covered stent applicable to vascular branches according to Embodiment 3 of the present application.
FIG. 11a-11d are schematic diagrams illustrating the operation process of a covered stent system applicable to vascular branches according to Embodiment 3 of the present application.
FIG. 12a and 12b are schematic diagrams illustrating another embodiment of the structure of a covered stent applicable to vascular branches according to Embodiment 3 of the present application.

### DETAILED DESCRIPTION OF THE INVENTION

The technical solutions and advantages brought by the technical solutions of the present application are further described in detail below with reference to the accompanying drawings and specific embodiments. The components in the drawings are not necessarily drawn to scale, emphasis instead being placed upon illustrating the concepts of the present application.

In the various embodiments of the present application, well-known structures or materials involved in are not described in detail. Furthermore, those skilled in the art should understand that the following various embodiments are only used for illustration, but not for limiting the protection scope of the present application.

Before describing the specific embodiments of the present application in detail, the terms involved in the present application are explained as follows:
"Microporous area" refers to an area defined by a plurality of micropores provided on a membrane, the plurality of micropores form an array according to different rules, and one array is one microporous area. The arrangement rules of each array can be the same or different. Pore sizes and/or pore spacing of the micropores in each array can be the same or different.

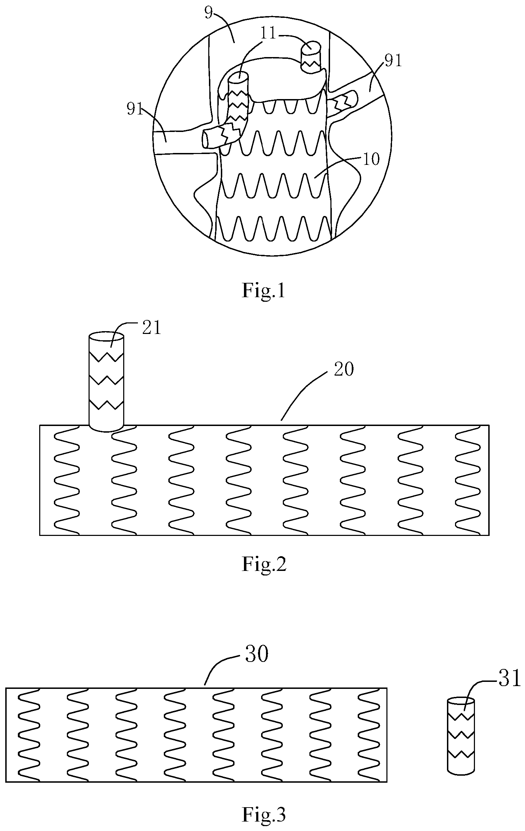

The "chimney" technique is a commonly used surgical technique for treating aortic lesions in the prior art. As shown in fig. 1, the specific operation steps are as follows: firstly, under the monitoring of Digital Subtraction Angiography (DSA), a main stent 10 and chimney stents 11 are respectively delivered into an aorta 9 and corresponding vascular branches 91 without release; then, after the positioning of each stent is determined by digital subtraction angiography, the main stent 10 in the aorta cavity is first released, and then the chimney stents 11 are released between the main stent 10 and the aorta wall. Blood supply channels flowing to the branches are extruded out by the supporting force of the chimney stents 11, and in order to facilitate blood flow into the chimney stents 11, when releasing, the chimney stents 11 need to exceed the front edge of the main stent 10 by at least 5mm.

The "branch stent" technique is a commonly used surgical technique for treating aortic lesions in the prior art. The structure of the branch covered stent is shown in fig. 2. The main stent 20 and the branch stent 21 in the branch covered stent are generally of an integral structure. The specific operation steps of the "branch stent" technique are as follows: firstly, under the monitoring of Digital Subtraction Angiography (DSA), a guide wire passing through the branch stent 21 is pushed into a corresponding branch vessel, and a guide wire passing through the main stent 20 is pushed to the aortic lesions, then the main stent 20 is conveyed to the aortic lesions along its inner guide wire and the branch stent is ensured to be pre-placed at the opening of the branch vessel along its inner guide wire. After the positions of the stents are adjusted and confirmed by radiography, the main stent 20 and the branch stent 21 are released to isolate the aortic lesions and maintain the blood flow of the branch vessel.

The "fenestration" technique is a commonly used surgical technique for treating aortic lesions in the prior art. Fig. 3 illustrates the structure of a covered stent for using in the in situ fenestration technique. In this structure, a main stent 30 and a branch stent 31 of a covered stent are of a separate structure. The specific operation steps of the covered stent are as follows: firstly, under the monitoring of Digital Subtraction Angiography (DSA), the main stent 30 of the covered stent is placed in the aorta cavity of the diseased aorta, and at this time, a membrane at the opening of a branch vessel can completely block the blood flow of the branch vessel; secondly, acupuncture (or laser, or radio frequency) and the like is used to perform membrane rupture and fenestration on the membrane at the opening of the branch vessel; then, dilating the original window by using a balloon; finally, the branch stent 31 is implanted after the dilation of the window is completed, and the window is supported so as to keep the branch blood flow unobstructed.

### Embodiment 1

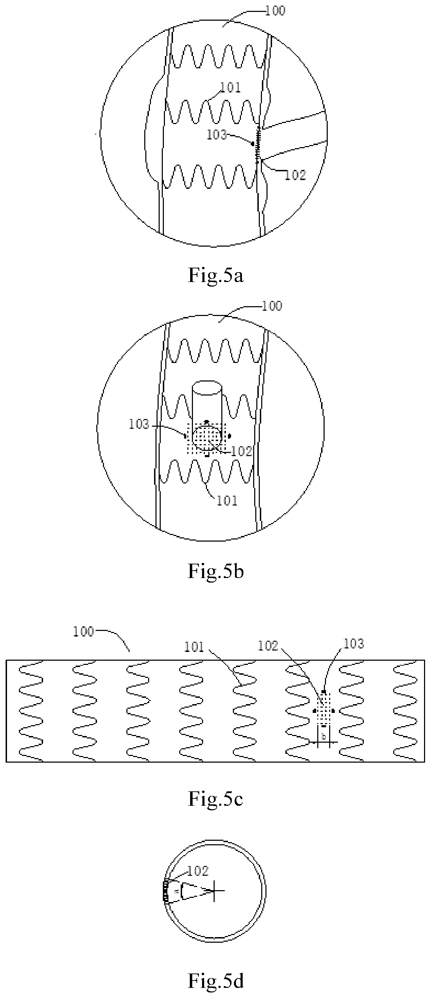

As shown in fig. 5a-5d, the present application provides a covered stent 100 for implantation at a vascular branch, the covered stent including a stent body 101 and a membrane fixedly connected to the stent body 101, wherein the stent body 101 is a straight cylindrical structure woven from nitinol wires which has sufficient supporting force. A microporous area 102 is provided on the membrane, and the microporous area 102 has a plurality of micropores which are arranged into an array with pore spacing ranging from 0.2mm to 2.0mm. The micropores are capable of allowing blood flow to pass through. During implantation of the covered stent 100, the microporous area 102 is used to identify branch region of the blood vessel and provide an access path for implantation of a branch stent.

In one embodiment, the diameters of the plurality of micropores provided in the microporous area 102 are equal, or the diameters of the micropores provided in the middle portion of the microporous areas 102 are larger than those provided in the edge portion of the microporous areas 102. The diameters of the micropores are in the range of 0.1 mm to 0.6 mm. The microporous area 102 is arranged in a range of 15 degrees to 360 degrees along the circumferential direction of the covered stent 100. The length b of the microporous area 102 is in the range of 3 mm to 100 mm.

In one embodiment, the interval between the micropores arranged in the middle portion of the microporous area 102 is smaller than the interval between the micropores arranged in the edge portion of the microporous area 102 . The shape of the microporous area 102 include, but is not limited to, a circle, an ellipses, a square, a strip, and the like. The length of the microporous area 102, the diameters of the micropores and the arrangement of the micropores can be adjusted according to the diameter and position of the branch vessel at the lesions. Due to the limitations of the present application on the pore sizes and arrangement of the micropores, as well as the characteristics of high blood flow and high pressure in the aorta, for the micropores that are not located at the opening of the vascular branch attached to the blood vessel wall, after a period of time, blood will accumulated in the micropores causing the micropores to be permanently closed, so there is no need to worry about endoleak.

In one embodiment, a plurality of development marks 103 are provided on the edge of the microporous area 102 to indicate the position of the microporous area 102. The development marks 103 are attached to the edge of the microporous area 102 by precision sewing or riveting. The shapes of the development marks include, but are not limited to, circles, ovals, "8" shapes, and the like. The number of the development marks 103 is 2 to10. The material of the development marks 103 includes, but is not limited to, platinum, gold, tantalum metal, medical stainless steel, and the like. In the present application, a plurality of development marks 103 are arranged on the edge of the microporous area 102 to indicate the position of the microporous areas 102. The microporous area section of the covered stent can be placed at the opening of the branch vessel through the development marks 103 under the monitoring of Digital Subtraction Angiography (DSA), so that the covered stent will not block the blood flow of the branch vessel while treating aortic lesions. Moreover, the development marks 103 are also beneficial to the positioning of the branch stent, which can be released at the target position after passing through the microporous area 102 to establish blood supply channel of the vascular branch.

In a preferred embodiment, the main stent is partially covered, and the stent at the proximal end portion of the stent body is uncovered. In a preferred embodiment, the main stent is fully covered.

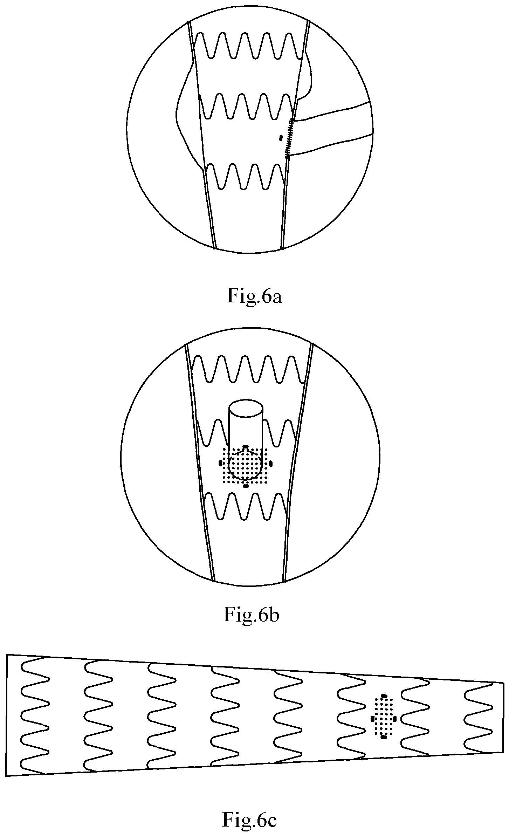

As shown in fig. 6a-fig. 6c, in another embodiment, the stent body 101 is a tapered structure formed by laser cutting, and the structure can adapt to the shape characteristics of the human blood vessels (such as carotid arteries) at the implantation site.

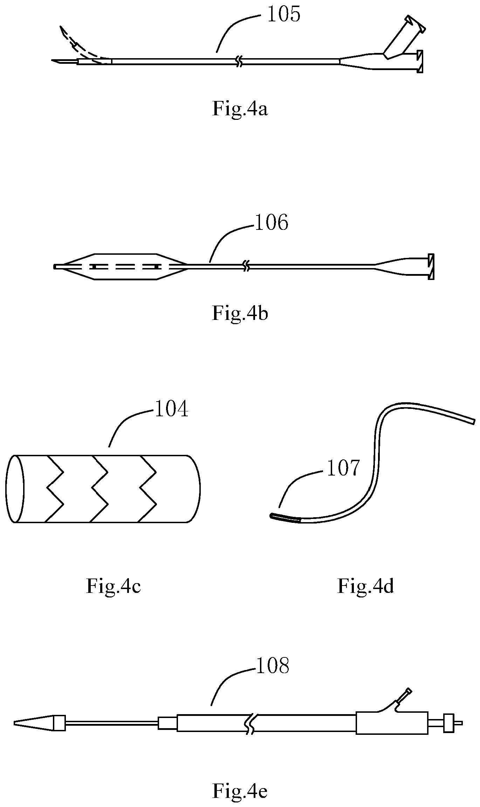

As shown in fig. 4a-fig. 4e, the present application also provides a covered stent system for implantation at a vascular branch, the system comprising a delivery system 108, a guide wire 107, and a covered stent provided in the delivery system, a membrane breaker 105, a dilatation balloon 106 and a branch stent 104. As shown in figs. 5a-5d, the covered stent 100 includes a stent body 101 and a membrane fixedly connected to the stent body 101. A microporous area 102 is provided on the membrane, and the microporous areas 102 has a plurality of micropores which are arranged into an array with pore spacing in the range of 0.2mm to 2.0mm. The micropores are capable of allowing blood flow to pass through. When the covered stent 100 is delivered to the target area by the delivery system and released, the blood flow through the micropores directs the microporous area 102 to be placed at the opening of the vascular branch, so the microporous area 102 can temporarily supply blood to the vascular branch. The membrane breaker 105 is delivered to the microporous area 102 by the delivery system, and the membrane breaker 105 is used to penetrate the microporous area 102 to provide an opening for the dilation balloon 106 to pass through. The dilation balloon 106 is used to further increase the size of the opening to facilitate the support of the branch stent 104 through the microporous area 102 at the vascular branch to establish the blood supply channel of the vascular branch.

In this embodiment, the delivery system 108 , the membrane breaker 105 , the dilation balloon 106 and the branch stent 104 may all use the structures commonly used in the prior art, and the detailed structures thereof will not be repeated herein again.

In this embodiment, the material of the stent body 101 includes, but is not limited to, nickel-titanium alloy, cobalt-chromium alloy, magnesium alloy, 316L stainless steel, metal tantalum, and polymer materials. The membrane is biocompatible, and is fixed to the stent body 101 by precision sewing or thermoforming. The material of the membrane includes but is not limited to polytetrafluoroethylene, polyester, polyethylene terephthalate, polyurethane esters, etc.

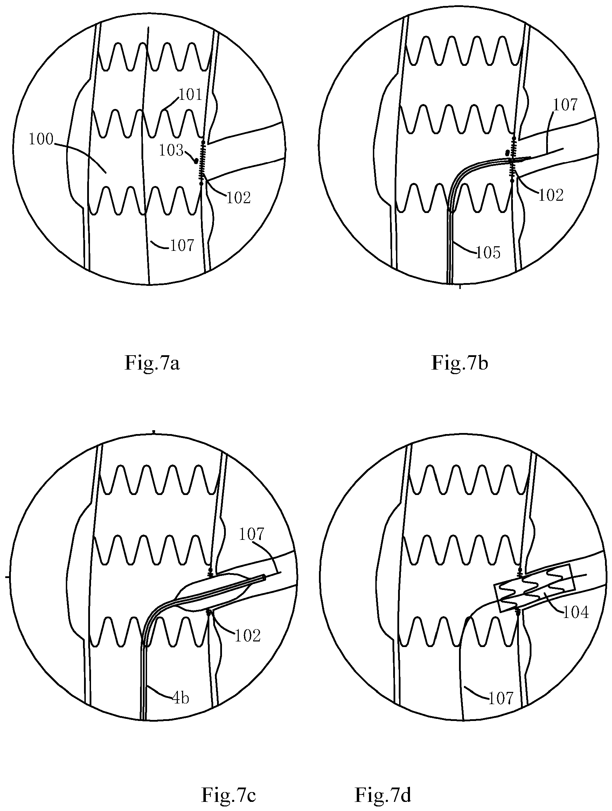

As shown in figs 7a-7d, the operation steps of the covered stent system of the present application are as follows:
Firstly, as shown in 7a, under the monitoring of digital subtraction angiography (DSA), the delivery system loaded with the covered stent 100 is delivered to the diseased blood vessel along the guide wire 107. Under the condition that the blood flow in the visible microporous area 102 passes through and/or under the indication of the development marks 103, the microporous area 102 are placed at the opening of the branch vessel, and the covered stent is released after accurately aligning. At this time, the covered stent not only covers the location of the arterial lesions, but also ensures the blood supply of the branch vessel due to the permeability of the microporous area 102 to blood flow.
Secondly, as shown in fig. 7b, the membrane breaker 105 is transported along the guide wire 107 to the opening of the branch vessel where the microporous area 102 is located, and the membrane perforation is performed to form an opening, and then the membrane breaker 105 is withdrawn after completion. Since the presence of the microporous area 102 reduces the transmembrane puncture force, the transmembrane puncture also becomes easier.
Thirdly, as shown in fig. 7c, the dilation balloon is transported along the guide wire 107 to the opening to further increase the size of the opening. Since the window dilation is performed in the microporous area 102, the risk of membrane tear at the window caused by balloon dilation is reduced to a certain extent.
Fourthly, as shown in fig. 7d , the branch stent 104 is implanted along the guide wire 107 at the opening of the branch vessel to support and stabilize the window, so as to keep the blood flow of the branch unobstructed.

It can be seen from the above description that the present application does not adopt the "chimney" technique that is prone to endoleak. The covered stent and covered stent system provided by the present application are provided with a microporous area on the membrane, and the microporous area has a plurality of micropores, which are capable of allowing blood flow to pass through. During the implantation of the covered stent, the microporous area can identify the vascular branch area and temporarily supply blood to the vascular branch, so the operation is simple and does not require establishing extracorporeal circulation, the operation time is short and the difficulty is low. Moreover, a plurality of micropores arranged in the microporous area are arranged into an array with pore spacing in the range of 0.2 mm to 2.0 mm, and the diameters of the micropores in the range of 0.1 mm to 0.6 mm. The setting of the micropore diameters and pore spacing can not only ensure the blood supply of the branch vessel, but also prevent the membrane from tearing during the puncture of the membrane breaker. Even if the dilation balloon is further dilated, it only changes the size of the adjacent micropores in the microporous area, but will not tear the membrane. Therefore, the microporous area can provide a good access indication and access window for the implantation of the branch stent, which not only solves the problems of difficult positioning and operation of the existing "branch stent" technique, but also solves the problems of serious surgical complications and complex surgery of the "fenestration" technique.

### Embodiment 2

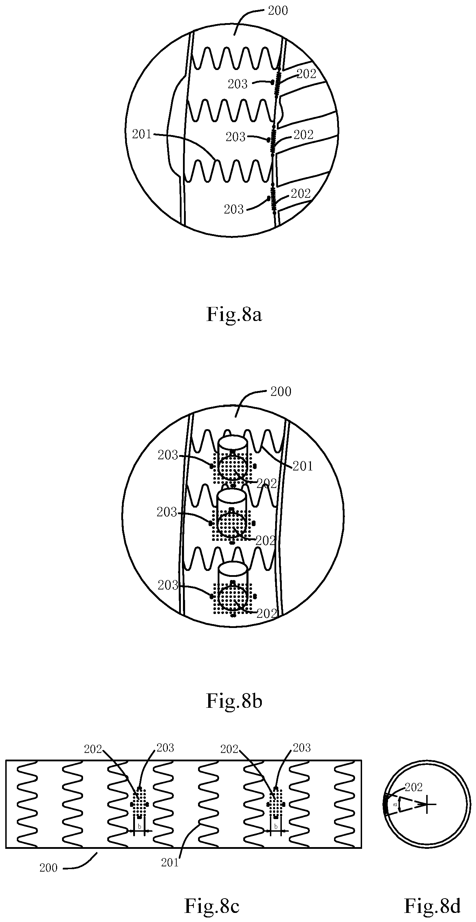

As shown in figs. 8a to 8d , a covered stent 200 for implantation at vascular branches, includes a stent body 201 and a membrane fixedly connected to the stent body 201. The stent body 201 is a straight cylindrical structure woven from nitinol wires and has sufficient supporting force. The difference between this Embodiment and Embodiment 1 is that a plurality of microporous areas 202 are provided on the membrane, which can be respectively placed at different branch vessel openings. This structure is suitable for the situation where there are multiple branch vessels at the lesion location. The setting of the pore diameters and the pore spacing of the plurality of micropores in the microporous areas 202 can be adjusted correspondingly according to the diameters and position of the branch vessels at the lesion. In one embodiment, a plurality of development marks 203 are provided on the edges of the plurality of microporous areas 202 to indicate the positions of the microporous areas 102.

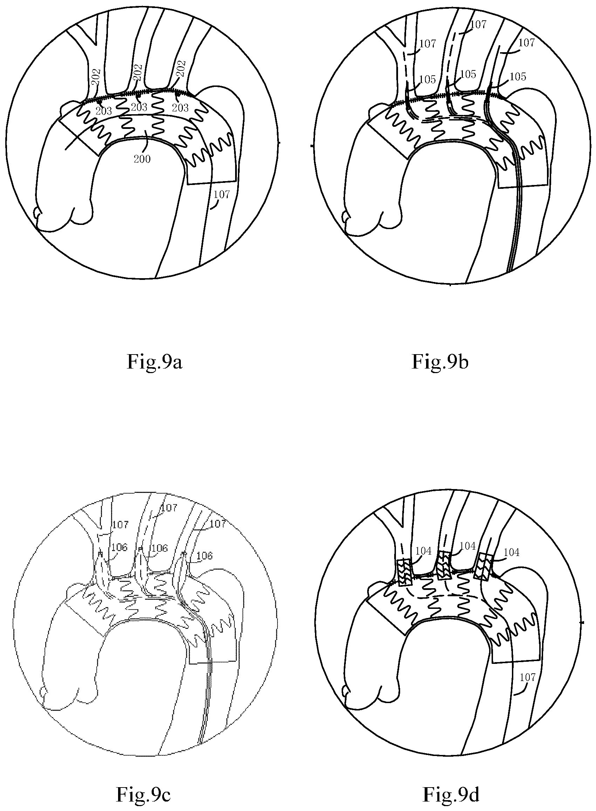

Figs 9a-9d show the operation steps of the covered stent system of this embodiment. The difference between this Embodiment and Embodiment 1 is that multiple microporous areas 202 need to be placed at different branch vessel openings, respectively to ensure the accuracy of the alignment and smooth blood flow of each branch vessel, and then repeat steps 2 to 4 several times respectively.

### Embodiment 3

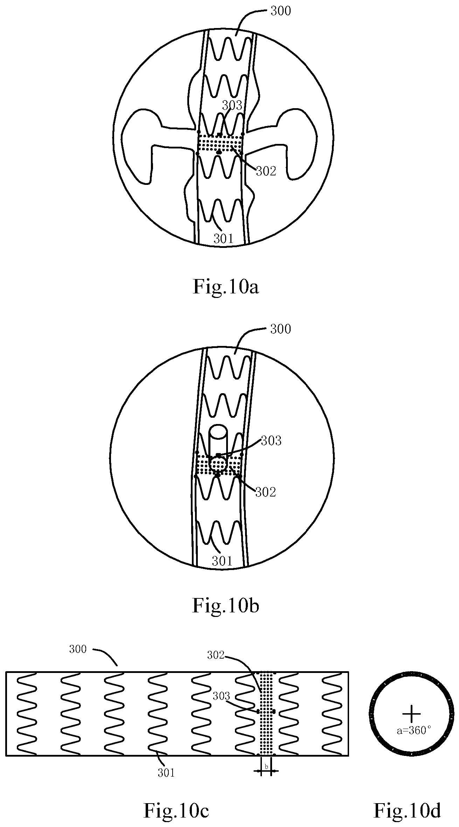

As shown in figs. 10a-10d, a covered 300 for implantation at vascular branches, includes a stent body 301 and a membrane fixedly connected to the stent body 301. The stent body 301 is a straight cylindrical structure woven from nitinol wires, which has sufficient supporting force. The difference between this embodiment and Embodiment 1 is that a certain area of the membrane is provided with micropores 360 degree along the circumferential direction of the stent body 301, that is, a microporous area 302 is provided 360 degree along the circumferential direction of the covered stent. The structure of the covered stent 300 is suitable for the case where there are multiple branch vessels in the same radial direction at the lesion location, such as renal artery vascular lesions.

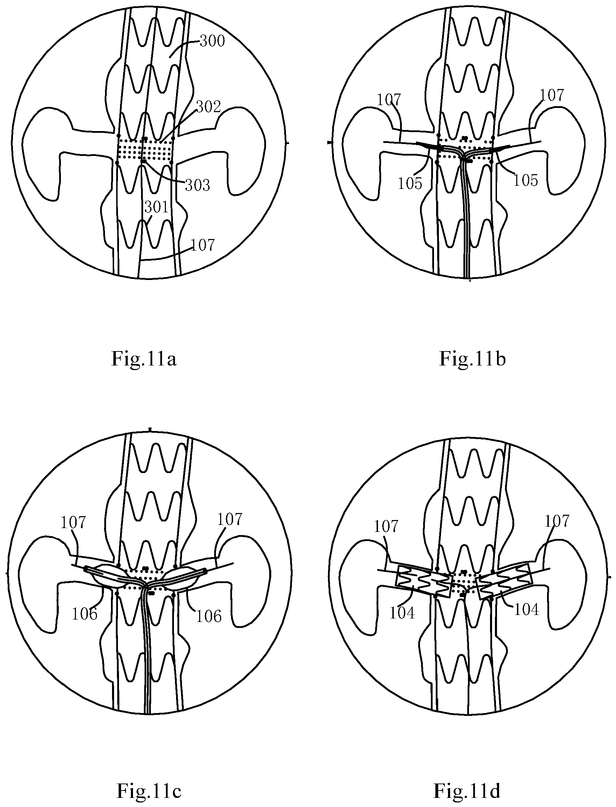

Figs 11a-11d show the operation steps of the covered stent system of this embodiment. The difference between this Embodiment and Embodiment 1 is that development marks 303 are provided on the edge of the microporous area 302. Under the indication of the development marks 303, the microporous area 302 is placed at the opening of the branch vessels. At this time, it is not necessary to consider the radial position of the microporous area 302. The membrane is provided with micropores at 360 degrees in a certain region, which can not only adapt to the situation that there are multiple branch vessels in the same radial direction at the lesion location, but also reduce the difficulty for the operator to align the microporous area 302 with the openings of the branch vessels. In addition, due to the limitation of the micropore diameters and the characteristics of fast blood flow and high pressure in the arteries, after a period of time, blood accumulation will occur in the micropores that are not located at the openings of the vascular branches in the same radial direction, resulting in permanent closure of the micropores, so there is no need to worry about endoleak.

### Embodiment 4

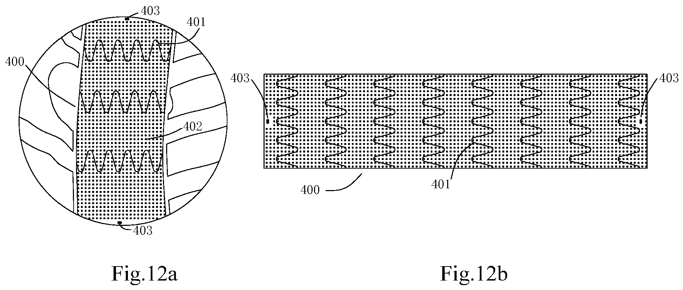

As shown in fig. 12a and fig. 12b, a covered stent 400 for implantation at vascular branches, includes a stent body 401 and a membrane fixedly connected to the stent body 401. The stent body 401 is a straight cylindrical structure woven from nitinol wires and has sufficient supporting force. Different from Embodiment 1, the covered stent in this embodiment is provided with a microporous area 402 on the entire membrane. The covered stent involved in this embodiment is suitable for cases with complex branches and numerous branches in aortic lesions, and the structure will not block the blood supply of the branch vessels.

In this embodiment, the difference of the operation steps between this embodiment and Embodiment 1 is that the two ends of the covered stent are provided with development marks 403. Under the indication of the development marks 403, the covered stent 400 is placed at the aortic lesions through a delivery system, subsequent operations such as hole breaking, dilating, and implantation of branch stents can be performed. Since there are micropores on the entire membrane, there is no problem of aligning the microporous area 403 with the openings of the branch vessels, so there is no need to worry about the membrane blocking the branch vessels. In addition, due to the limitation of the micropore diameters and the characteristics of high blood flow rate and high pressure in the aorta, for the micropores that are not located at the openings of the vascular branches, after a period of time, blood accumulation will appear in the micropores, causing the micropores to be permanently closed, so there is no need to worry about endoleak.

In the specific application process, the appropriate number and range of microporous areas can be set according to the anatomical structure of the lesion area, and the microporous areas can be set within the range of 15 degrees to 360 degrees along the circumferential direction of the covered stent.

## Claims

1. A covered stent for implantation at vascular branches, comprising a stent body (101, 201, 301, 401) and a membrane fixedly connected to the stent body (101, 201, 301, 401), **characterized in that** one or more microporous areas (102, 202, 302, 402) are provided on the membrane, and each of the one or more microporous areas (102, 202, 302, 402) has a plurality of micropores which are arranged into an array with pore spacing ranging from 0.2mm to 2.0mm, and the micropores are capable of allowing blood flow to pass through, and during implantation of the covered stent (100, 200, 300, 400), each of the one or more microporous areas (102, 202, 302, 402) is used to identify a branch region of a blood vessel and provide an access path for implantation of a branch stent (104) and each of the one or more microporous areas (102, 202, 302, 402) is arranged in a range of 15 degrees to 360 degrees along the circumferential direction of the covered stent (100, 200, 300, 400);
wherein the diameters of the micropores are in the range 0.1 mm to 0.6 mm, and wherein a plurality of development marks (103, 203, 303, 403) are provided on the edge of each of the one or more microporous areas (102, 202, 302, 402) to indicate the position of the respective microporous area (102, 202, 302, 402) and a material of the development marks (103, 203, 303, 403) includes platinum, gold, tantalum metal or medical stainless steel.

2. The covered stent for implantation at vascular branches according to claim 1, wherein a length of each of the one or more microporous areas (102, 202, 302, 402) is in the range of 3mm to 100mm, and the length is taken along an axis parallel to the longitudinal axis of the covered stent.

3. The covered stent for implantation at vascular branches according to claim 1, wherein the development marks (103, 203, 303, 403) are attached to the edge of the microporous areas (102, 202, 302, 402) by precision sewing or riveting.

## Patentansprüche

1. Ein Stenttransplantat zum Implantieren eines Gefäßastes, das die Stentkörperen (101, 201, 301, 401) und eine mit den Gerüstkörperen (101, 201, 301, 401) feste verbundene Schichtung umfasst, **dadurch gekennzeichnet, dass** eine oder mehrere Mikroporöse-Bereiche (102, 202, 302, 402) auf der Schichtung angeordnet sind und jeder der einen oder mehrere Mikroporöse-Bereiche (102, 202, 302, 402) eine Vielzahl von Mikroporöse aufweist, wobei die Mikroporen in einem Array mit einem Lochabstand im Bereich von 0,2 mm bis 2,0 mm angeordnet sind, die Mikroporen den Blutfluss leiten können, und während des Prozesses der Implantation der Stenttransplantate (100, 200, 300, 400) wird jede der eine oder mehrere Mikroporöse-Bereiche (102, 202, 302, 402) zur Identifizierung des Gefäßastbereichs und zur Bereitstellung eines Ansatzes für die Implantation des Aststents (104) verwendet; und jeder der einen oder mehrere Mikroporöse-Bereiche (102, 202, 302, 402) innerhalb des Bereichs von 15 Grad bis 360 Grad entlang der Umfangsrichtung der Stenttransplantate (100, 200, 300, 400) angeordnet sind;
wobei der Durchmesser der Mikroporen 0,1 mm bis 0,6 mm ist, wobei eine Vielzahl von Entwicklungsmarken (103, 203, 303, 403) an der Kante jedes der einen oder mehrerer Mikroporöse-Bereiche (102, 202, 302, 402) angeordnet sind, um die Position der jeweiligen Mikroporöse-Bereiche (102, 202, 302, 402) zu markieren, und die Materialien der Entwicklungsmarken (103, 203, 303, 403) Platin, Gold, Tantalmetall oder medizinischer Edelstahl umfassen.

2. Das Stenttransplantat zum Implantieren eines Gefäßastes nach Anspruch 1, **dadurch gekennzeichnet, dass** die Länge jeder der einen oder mehrerer Mikroporöse-Bereiche (102, 202, 302, 402) im Bereich von 3 mm bis 100 mm liegt und die Länge entlang einer Achse parallel zur Längsachse des Stenttransplantat gemessen wird.

3. Das Stenttransplantat zum Implantieren eines Gefäßastes nach Anspruch 1, **dadurch gekennzeichnet, dass** die Entwicklungsmarke (103, 203, 303, 403) mit dem Rand der Mikroporöse-Bereiche (102, 202, 302, 402) durch Präzisionsnaht oder Nieten verbunden sind.

## Revendications

1. Stent recouvert destiné à être implanté au niveau de branches vasculaires, comprenant un corps de stent (101, 201, 301, 401) et une membrane reliée de manière fixe au corps de stent (101, 201, 301, 401), **caractérisé en ce qu'**une ou plusieurs zones microporeuses (102, 202, 302, 402) sont disposées sur ladite membrane, chacune de la ou des zones microporeuses (102, 202, 302, 402) comporte une pluralité de micropores qui sont disposés en un réseau avec un espacement des pores allant de 0,2 mm à 2,0 mm, et les micropores sont capables de laisser passer le flux sanguin, et pendant l'implantation du stent recouvert (100, 200, 300, 400), chacune de la ou des zones microporeuses (102, 202, 302, 402) est utilisée pour identifier une région de branches vasculaires et fournir un chemin d'accès pour l'implantation d'un stent de branche (104) et chacune des une ou plusieurs zones microporeuses (102, 202, 302, 402) est disposée dans une plage de 15 degrés à 360 degrés le long de la direction circonférentielle du stent recouvert (100, 200, 300, 400);
dans lequel le diamètre des micropores est compris entre 0,1 mm et 0,6 mm, et dans lequel plusieurs marqueurs de visualisation (103, 203, 303, 403) sont prévues sur le bord de chacune des une ou plusieurs zones microporeuses (102, 202, 302, 402) pour indiquer la position de la zone microporeuse respective (102, 202, 302, 402) et un matériau des marqueurs de visualisation (103, 203, 303, 403) comprend du platine, de l'or, du tantale métallique ou de l'acier inoxydable médical.

2. Le stent recouvert destiné à être implanté au niveau de branches vasculaires selon la revendication 1, **caractérisé en ce que** la longueur de chacune de la ou des zones microporeuses (102, 202, 302, 402) est comprise entre 3 mm et 100 mm, et la longueur est mesurée le long d'un axe parallèle à l'axe longitudinal du stent recouvert.

3. Le stent recouvert destiné à être implanté au niveau de branches vasculaires selon la revendication 1, **caractérisé en ce que** les marqueurs de visualisation (103, 203, 303, 403) sont reliées au bord de la ou des zones microporeuses (102, 202, 302, 402) par manière de suture précise ou de rivetage.
